(19) Europäisches Patentamt
European Patent Office
Office européen des brevets

(11) Veröffentlichungsnummer: **0 031 029**
**A1**

(12) # EUROPÄISCHE PATENTANMELDUNG

(21) Anmeldenummer: **80107117.6**

(22) Anmeldetag: **17.11.80**

(51) Int. Cl.³: **A 01 N 43/50**
A 01 N 43/64, A 01 N 47/10
A 01 N 47/20, C 07 D 233/60
C 07 D 249/08

(30) Priorität: **30.11.79 DE 2948206**

(43) Veröffentlichungstag der Anmeldung:
**01.07.81 Patentblatt 81/26**

(84) Benannte Vertragsstaaten:
**AT BE CH DE FR GB IT LI LU NL SE**

(71) Anmelder: **BASF Aktiengesellschaft**
**Carl-Bosch-Strasse 38**
**D-6700 Ludwigshafen(DE)**

(72) Erfinder: **Rentzea, Costin, Dr.**
**Neuenheimer Landstrasse 72**
**D-6900 Heidelberg(DE)**

(72) Erfinder: **Sauter, Hubert, Dr.**
**Neckarpromenade**
**D-6800 Mannheim(DE)**

(72) Erfinder: **Pommer, Ernst-Heinrich, Dr.**
**Berliner Platz 7**
**D-6703 Limburgerhof(DE)**

(54) **Gamma-Azolyl-Verbindungen enthaltende Fungizide und deren Verwendung.**

(57) Die vorliegende Ammeldung betrifft Fungizide, welche als Wirkstoff eine Verbindung der Formel

$$\begin{array}{l} R^1-\underset{\displaystyle Az}{\underset{|}{C}}H-CH_2-\underset{\displaystyle O-R^3}{\underset{|}{C}}H-R^2 \end{array}$$

worin
$R^1$, $R^2$, $R^3$ und Az die in den Ansprüchen angegebene Bedeutung besitzen, enthalten sowie deren Verwendung.

EP 0 031 029 A1

# γ-Azolyl-Verbindungen enthaltende Fungizide und deren Verwendung

Die vorliegende Erfindung betrifft neue Fungizide, die γ-Azolyl-Verbindungen enthalten, sowie Verfahren zur Bekämpfung von phytopatogenen Pilzen mit den Fungiziden.

Es ist bekannt, daß das 1-(2-(2,4-Dichlorphenyl-2-(2--propenyloxy)-ethyl)-1-H-imidazol eine gute fungizide Wirksamkeit zeigt (DE-OS 20 63 857). Seine Wirkung ist bei niedrigen Aufwandmengen nicht immer ausreichend. Ferner ist seine fungizide Wirkung mit einer hohen Phytotoxizität verbunden, so daß bei den für die Bekämpfung von Rostpilzen notwendigen Konzentration auch Kulturpflanzen geschädigt oder in ihrem Wachstum gehemmt werden. Aus diesen Gründen ist es für den Gebrauch als Pflanzenschutzmittel zur Bekämpfung von phytopathogenen Pilzen nicht immer und nicht bei allen Pflanzenarten geeignet.

Es ist weiter aus der EP-OS 00 10 287 bekannt, daß bestimmte γ-Azolyl-Verbindungen gute wachstumsregulierende Mittel sind.

Es wurde nun gefunden, daß die in der genannten EP-OS beschriebenen Substanzen sowie eine Reihe sehr ähnlicher Verbindungen eine sehr gute fungizide Wirksamkeit bei ausgezeichneter Pflanzenverträglichkeit besitzen.

Die Erfindung betrifft daher Fungizide, die γ-Azolyl-Verbindungen der Formel I

$$R^1\text{-}\underset{\displaystyle Az}{CH}\text{-}CH_2\text{-}\underset{\displaystyle O\text{-}R^3}{CH}\text{-}R^2 \qquad (I)$$

worin

$R^1$ und $R^2$ gleich oder verschieden sind und Alkyl oder Alkenyl mit bis zu 8 C-Atomen, Cycloalkyl mit bis zu 7 C-Atomen, Furanyl, Thienyl, Naphthyl oder einen gegebenenfalls durch Fluor, Chlor, Brom, Nitro, Trifluormethyl oder Alkyl, Alkoxy oder Alkenyl mit bis zu 8 C-Atomen substituierten Phenylrest bedeuten,

$R^3$ einen gegebenenfalls durch Chlor oder Alkoxy substituierten Alkylrest mit bis zu 10 C-Atomen; einen gegebenenfalls durch Chlor substituierten Alkenylrest mit bis zu 5 C-Atomen; einen Alkinylrest mit bis zu 5 C-Atomen; einen gegebenenfalls durch Fluor, Chlor, Brom, Nitro, Trifluormethyl oder Alkyl, Alkoxy oder Alkenyloxy mit bis zu 4 C-Atomen substituierten Benzylrest; einen $-CO-R^4$-Rest, worin $R^4$ einen gegebenenfalls durch Halogen, Alkoxy, Oxo (= O) oder Carboxyalkyl substituierten Alkylrest mit bis zu 6 C-Atomen oder einen aromatischen Rest bedeutet; oder einen $-CO-NH-R^5$-Rest, worin $R^5$ einen Alkylrest mit bis zu 4 C-Atomen oder einen aromatischen Rest bedeutet, darstellt und

Az einen 1-Imidazolyl-, 1,2,4-Triazolyl- oder 1,2,3-Triazolylrest bedeutet,

sowie deren für Pflanzen verträgliche Salze und Metallkomplexe enthalten.

Die $\gamma$-Azolyl-Verbindungen sind zum großen Teil aus der EP-OS 00 10 287 bekannt. Zusätzlich zu den dort genannten Verbindungen 1-94 wurden folgende Verbindungen hergestellt:

| Beispiel Nr. | $R^1$ | $R^2$ | $R^3$ | Az | Fp/°C | IR Film [$cm^{-1}$] |
|---|---|---|---|---|---|---|
| 95 | 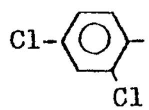 | $(CH_3)_3C-$ | $-CO-CH_3$ | 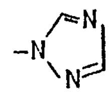 | Harz | 2960, 2870, 1730, 1588, 1560, 1500, 1470, 1370, 1240, 1140, 1020, 865, 855, 682 |
| 96 | 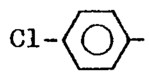 | $(CH_3)_3C-$ | $-COCH_2CH_3$ | " | Harz | 2980, 1730, 1597, 1495, 1370, 1278, 1185, 1142, 1095, 1010, 850, 810, 740, 682 |
| 97 | 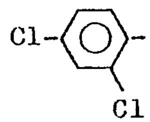 | $(CH_3)_3C-$ | $-COCH_2CH_3$ | " | Harz | 2960, 1730, 1590, 1502, 1475, 1368, 1275, 1180, 1082, 1008, 865, 854, 825 |
| 98 | 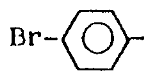 | $(CH_3)_3C-$ | $-CO-CH_3$ | " | Harz | 2963, 1730, 1500, 1490, 1372, 1275, 1240, 1205, 1140, 1075, 1012, 960, 805, 685 |
| 99 | 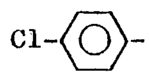 | $(CH_3)_3C-$ | $-CO-C_6H_5$ | " | Harz | 2955, 1710, 1596, 1490, 1366, 1270, 1136, 1110, 1090, 1012, 710, 676, 650 |
| 100 | 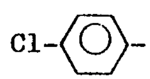 | $(CH_3)_3C-$ | $-CO-C_3H_7-n$ | " | Harz | 2960, 1725, 1490, 1365, 1270, 1175, 1136, 1090, 1014, 955, 803, 678, 650 |
| 101 | 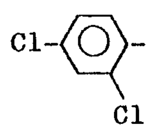 | $-CH(CH_3)-C_2H_5$ | $-CO-CH_3$ | " | Harz | 2952, 2865, 1730, 1585, 1497, 1465, 1370, 1270, 1230, 1135, 1015, 860, 818, |

| Beispiel Nr. | $R^1$ | $R^2$ | $R^3$ | Az | Fp/°C | IR Film [$cm^{-1}$] |
|---|---|---|---|---|---|---|
| 102 | 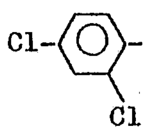 | $-\underset{CH_3}{CH}-C_2H_5$ | $-COCH_2CH_3$ | 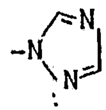 | Harz | 2950, 1730, 1585, 1496, 1460, 1270, 1180, 1135, 1000, 860, 820, 676 |
| 103 | 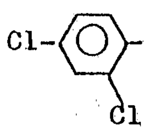 | $(CH_3)_3C-$ | $-CO-C_6H_5$ | " | Harz | 2950, 1710, 1582, 1495, 1468, 1362, 1268, 1106, 820, 708, 677 |
| 104 | 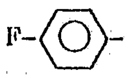 | 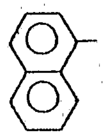 | $-CO-CH_3$ | " | Harz | 2945, 1735, 1600, 1494, 1365, 1270, 1220, 1130, 1020, 832, 795, 775, 675 |
| 105 | 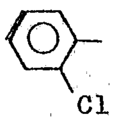 | $-\underset{CH_3}{CH}-C_2H_5$ | $-CO-CH_3$ | " | Harz | 2972, 1730, 1498, 1440, 1370, 1270, 1235, 1137, 1035, 1020, 752, 680, 658 |
| 106 | 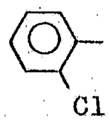 | $-\underset{CH_3}{CH}-C_2H_5$ | $-COCH_2CH_3$ | " | Harz | 2975, 2870, 1728, 1497, 1458, 1270, 1182, 1136, 1004, 750, 678, 658 |
| 107 | 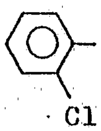 | $-\underset{CH_3}{CH}-C_2H_5$ | $-CO-C_6H_5$ | " | Harz | 2970, 1712, 1495, 1452, 1364, 1270, 1137, 1108, 750, 678, 660 |
| 108 | 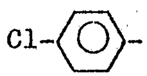 | 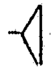 | $-CH_3$ | " | Harz | 3000, 2925, 1595, 1495, 1412, 1375, 1200, 1180, 1110, 1090, 1016, 880, 855, 830, 738, 680, 656 |

| Beispiel Nr. | $R^1$ | $R^2$ | $R^3$ | Az | Fp/°C | IR Film [$cm^{-1}$] |
|---|---|---|---|---|---|---|
| 109 | 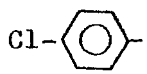 | 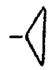 | Allyl | 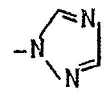 | Harz | 3000, 2920, 1596, 1498, 1428, 1270, 1196, 1140, 1084, 1015, 960, 926, 840, 743, 682, 658, 570 |
| 110 | $C_6H_5-$ | 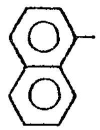 | Allyl | " | Harz | 2970, 2860, 1595, 1500, 1450, 1340, 1274, 1200, 1138, 1066, 1005, 800, 780, 766, 703, 680 |
| 111 | $C_6H_5-$ | 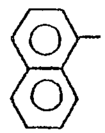 | $-C_5H_{11}-$ | " | Harz | 2940, 1598, 1498, 1345, 1272, 1196, 1100, 1005, 956, 855, 800, 780, 705, 680, 665, |
| 112 | $(CH_3)_3C-$ | 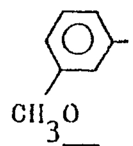 | $n-C_3H_7-$ | " | Harz | 2960, 2870, 1592, 1578, 1495, 1478, 1362, 1260, 1136, 1085, 1040, 952, 872, 786, 710, 696, 678 |
| 113 | $(CH_3)_3C-$ | 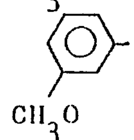 | $n-C_4H_9-$ | " | Harz | 2958, 2865, 1592, 1579, 1495, 1478, 1426, 1260, 1135, 1085, 952, 870, 785, 710, 695, 680, 660 |
| 114 | $(CH_3)_3C-$ | 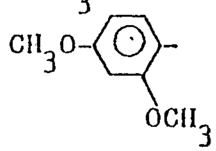 | $n-C_3H_7-$ | " | Harz | 2958, 1605, 1580, 1495, 1455, 1362, 1295, 1270, 1200, 1150, 1112, 1085, 1034, 830, 678 |
| 115 | $(CH_3)_3C-$ | 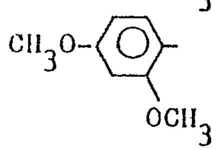 | $n-C_4H_9-$ | " | Harz | 2950, 1604, 1578, 1492, 1456, 1295, 1266, 1200, 1150, 1112, 1080, 1030, 950, 830, 678 |

| Beispiel Nr. | $R^1$ | $R^2$ | $R^3$ | Az | Fp/°C | IR Film [$cm^{-1}$] |
|---|---|---|---|---|---|---|
| 116 | $(CH_3)_3C-$ | 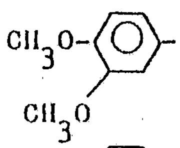 | $n-C_3H_7-$ | 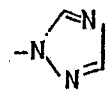 | Harz | 2952, 2865, 1596, 1585, 1500, 1466, 1258, 1232, 1132, 1084, 1022, 951, 852, 808, 760, 678 |
| 117 | $(CH_3)_3C-$ | 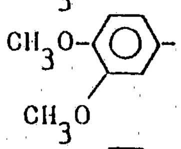 | $n-C_4H_9-$ | " | Harz | 2960, 2870, 1600, 1588, 1500, 1458, 1365, 1255, 1232, 1086, 1025, 952, 857, 808, 762, 680 |
| 118 | $(CH_3)_3C-$ | 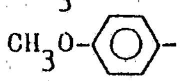 | $n-C_4H_9-$ | " | Harz | 2950, 1603, 1492, 1458, 1360, 1240, 1134, 1085, 1030, 951, 830, 680, 576 |
| 119 | $(CH_3)_3C-$ | 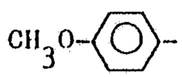 | $Cl-(CH_2)_4$ | " | Harz | 2955, 2863, 1600, 1500, 1362, 1240, 1165, 1088, 1030, 950, 832, 680 |
| 120 | $(CH_3)_3C-$ | 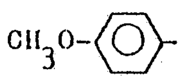 | $n-C_5H_{11}-$ | " | Harz | 2948, 1600, 1573, 1500, 1490, 1360, 1265, 1236, 1192, 1132, 1084, 948, 828, 676 |
| 121 | $(CH_3)_3C-$ | 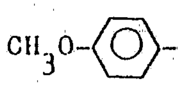 | $n-C_6H_{13}-$ | " | Harz | 2950, 2826, 1600, 1500, 1458, 1361, 1263, 1238, 1165, 1132, 1085, 1030, 950, 830, 778 |
| 122 | $(CH_3)_3C-$ | 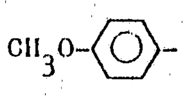 | $n-C_8H_{17}-$ | " | Harz | 2948, 2825, 1598, 1498, 1455, 1292, 1262, 1235, 1190, 1130, 1084, 1025, 948, 826, 675 |

| Beispiel Nr. | $R^1$ | $R^2$ | $R^3$ | Az | Fp/°C | IR Film [$cm^{-1}$] |
|---|---|---|---|---|---|---|
| 123 | $(CH_3)_3C-$ | 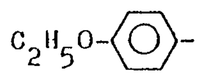 | $n-C_3H_7$ | 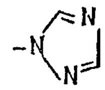 | Harz | 2952, 1597, 1500, 1468, 1360, 1262, 1236, 1162, 1106, 1080, 1040, 830, 676 |
| 124 | $(CH_3)_3C-$ | 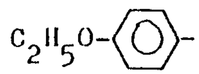 | $n-C_4H_9$ | " | Harz | 2950, 1597, 1497, 1467, 1263, 1232, 1162, 1130, 1105, 1082, 1040, 830, 675 |
| 125 | $(CH_3)_3C-$ | 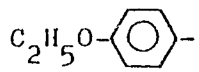 | $n-C_5H_{11}$ | " | Harz | 2950, 1595, 1496, 1466, 1358, 1233, 1162, 1106, 1082, 1040, 830, 675 |
| 126 | $(CH_3)_3C-$ | 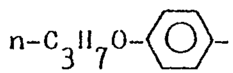 | $n-C_3H_7-$ | " | Harz | 2948, 1596, 1495, 1358, 1262, 1232, 1130, 1080, 825, 676 |
| 127 | $(CH_3)_3C-$ | 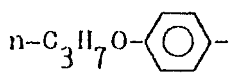 | $n-C_4H_9-$ | " | Harz | 2952, 1600, 1500, 1468, 1360, 1262, 1235, 1163, 1132, 1085, 1002, 828, 678 |
| 128 | $(CH_3)_3C-$ | 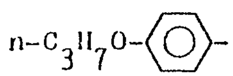 | $n-C_5H_{11}-$ | " | Harz | 2950, 2920, 1599, 1500, 1467, 1360, 1263, 1235, 1084, 1002, 828, 679 |
| 129 | $(CH_3)_3C-$ | 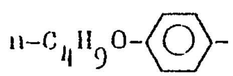 | $n-C_3H_7-$ | " | Harz | 2956, 2862, 1598, 1498, 1466, 1358, 1262, 1235, 1080, 828, 678 |
| 130 | $(CH_3)_3C-$ | 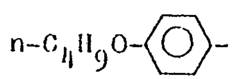 | $n-C_4H_9-$ | " | Harz | 2948, 2910, 2860, 1596, 1498, 1465, 1358, 1235, 1130, 1084, 825, 675 |

1-(4-Chlor-1-butoxy)-1-(4-methoxyphenyl)-3-(1,2,4-triazolyl-(1))-4,4-dimethylpentan,
1-(5-Chlor-1-pentyloxy)-1-(4-methoxyphenyl)-3-(1,2,4-triazolyl-(1))-4,4-dimethylpentan,
1-Methoxy-1-(4-ethoxyphenyl)-3-(1,2,4-triazolyl-(1))-4,4-dimethylpentan,
1-Ethoxy-1-(4-ethoxyphenyl)-3-(1,2,4-triazolyl-(1))-4,4-dimethylpentan,

Die neuen Verbindungen lassen sich nach dem in der EP-OS 00 10 287 angegebenen Verfahren herstellen.

Falls gewünscht, werden die Verbindungen nach bekannten Verfahren in ihre für Pflanzen verträglichen Salze oder in ihre Metallkomplexe überführt.

Zur Salzbildung eignen sich beispielsweise: Salzsäure, Bromwasserstoffsäure, Schwefelsäure, Salpetersäure, Phosphorsäure, Essigsäure oder Dodecylbenzolsulfonsäure. Die Wirksamkeit der Salze geht auf das Kation zurück, so daß die Wahl des Anions beliebig ist.

Metallkomplexe bilden sich durch Anlagerung der neuen Verbindungen an die Kationen von Metallsalzen. Besonders eignen sich hierfür Kupfer(II)-chlorid, Kupfer(II)-sulfat, Kupfer-(II)-nitrat, Zink(II)-chlorid, Eisen(III)-chlorid.

Die hier beschriebenen γ-Azolyl-Verbindungen und ihre Salze und Metallkomplexverbindungen zeigen eine erheblich breitere fungizide Wirkung und eine überlegene Pflanzenverträglichkeit als das bekannte 1-(2-(2,4-Dichlorphenyl)-2-(2-propenyloxy)-ethyl)-1H-imidazol. Die Verbindungen der allgemeinen Formel I zeigen ferner auch eine starke fungitoxische Wirksamkeit auch gegen phytopathogene Pilze aus der Klasse der Phycomyceten. Auch hier zeigen die

erfindungsgemäßen γ-Azolylverbindungen eine erhebliche breitere fungizide Wirksamkeit und eine hervorragende systemische und curative Wirkungsweise als die bekannten Mangan-, bzw. Zink-ethylen-bis-dithiocarbamidate und das N-Trichlormethylthio-phthalimid.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Apfelbaum, Gurken, Bohnen, Sonnenblumen, Tabak, Salat, Zwiebeln, Hopfen, Tomaten, Kartoffeln, Paprika, Kaffee, Zuckerrohr, Weinrebe, Erdbeeren sowie Zierpflanzen im Gartenbau.

Die neuen Verbindungen sind insbesondere geeignet zur Bekämpfung folgender Pflanzenkrankheiten:

Erysiphe graminis (echter Mehltau) in Getreide,
Puccinia Arten (Getreiderost),
Hemileia vastatrix (Kaffeerost),
Ustilago Scitaminea (Zuckerrohrbrand),
Phytophthora infestans an Tomaten und Kartoffeln,
Phytophthora garasitica an Erdbeeren,
Phytophthora cactorum an Äpfeln,
Pseudoperonospora cubensis an Gurken,
Pseudoperonospora humuli an Hopfen,
Peronospora destructor an Zwiebeln,
Peronospora sparsa an Rosen,
Peronospora tabacina an Tabak,
Plasmopara viticola an Reben,
Plasmopara halstedii an Sonnenblumen,
Sclerospora macrospora an Mais,
Bremia lactucae an Salat,
Mucor mucedo an Früchten,
Rhizopus nigricaus an Rüben,

Botrytis cinerae an Rebe, Erdbeeren,
Uncinula necator,
Sphaeroteca fuliginea,
Erysiphe cichoracearum,
Podosphaera leucotricha.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzenteile möglich ist.

Die neuen Wirkstoffe werden beispielsweise in Form von direkt versprühbaren Lösungen, Pulvern, Suspensionen, auch hochprozentige wäßrige, ölige oder sonstige Suspensionen doer Dispersionen, Emulsionen, Öldispersionen, Pasten, Stäubemitteln, Streumitteln, Granulaten durch Versprühen, Vernebeln, Verstäuben, Verstreuen oder Gießen angewendet. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfranktionen von mittlerem bis hohem Siedepunkt, wie Kerosin oder Dieselöl, ferner Kohlenteeröle usw., sowie Öle pflanzlichen oder tierischen Ursprungs, aliphatische, cyclische

und aromatische Kohlenwasserstoffe, z.B. Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden, Zur Herstellung von Emulsionenb, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkalarylpolyetheralkohole, Isotridecylalkohol, Fettalko-

holethylenoxid-Kondensate, ethoxyliertes Rizinusöl, Poloxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 5 kg Wirkstoff oher mehr je ha.

Beispiele für solche Zubereitungen sind:

I. Man vermischt 90 Gewichtsteile der Verbindung des Beispiels 1 der EP-OS 00 10 287 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 2 der EP-OS 00 10 287 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung des Beispiels 3 der EP-OS 00 10 287 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile der Verbindung des Beispiels 5 der EP-OS 00 10 287 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile der Verbindung des Beispiels 6 der EP-OS 00 10 287 werden mit 3 Gewichtsteilen des Natriumsalzes der Diisobutylnaphthalin- -sulfonsäure,

17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichts- teilen Wasser erhält man eine Spritzbrühe, die 0,1 Gew.% des Wirkstoffs enthält.

VI. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 2 der EP-OS 00 10 287 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 der EP-OS 00 10 287 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

IX. 20 Teile der Verbindung des Beispiels 4 der EP-OS 00 10 287 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen

eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion.

Die folgende Liste von Fungiziden, mit denen die erfindungs-gemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie Ferridimethyldithiocarbamat, Zinkdimethyldithiocarbamat, oder Zinkethylenbisdithiocarbamat,
Tetramethylthiuramidsulfide,
Zink-(N,N'-propylen-bis-dithiocarbamat),
Ammoniak-Komplex von Zink-(N,N'-propylen-bis-dithiocarbamat) und N,N'-Propylen-bis(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
4-(2-Chlorphenylhydrazono)-3-methyl-5-isoxazolon,
Pyridin-2-thio-1-oxid,
8-Hydroxychinolin bzw. dessen Kupfersalz,

2,3-Dihydro-5-carboxanilido-6-methyl-1,4-oxathiin-4,4-dioxid, 2,3-Dihydro-5-carboxanilido-6-methyl-1,4-oxathiin-2-(furyl(2))-benzimidazol,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-pyridinmethanol,
1,2-Bis-(3-ethoxycarbonyl-2-thioureido)-benzol,
1,2-Bis-(3-methoxycarbonyl-2-thioureido)-benzol

und weitere Substanzen, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid 2,5-Dimethyl-furan-3-carbonsäureanilid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol.

Die folgenden Beispiele A-E zeigen die biologische Wirkung der neuen Substanzen. Als Vergleichssubstanzen dienten die bekannten Wirkstoffe Imazalil (A), Folpet (B) und Zineb (C). Die Substanzen der Beispiele 1-94 sind der EP-OS 00 10 287 zu entnehmen.

Beispiel A

Blätter von in Töpfen gewachsenen Gerstenkeimlingen werden mit wäßrigen Emulsionen aus 80 % (Gew.%) Wirkstoff und 20 % Emulgiermittel besprüht und nach dem Antrocknen des Spritzbelages mit Oidien (Sporen) des Gerstenmehltaus (Erysiphe graminis var. hordei) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

| Wirkstoff des Beispiels Nr. | Befall der Blätter nach Spritzung mit x %iger Wirkstoffbrühe x = 0,025 | 0,012 | 0,006 |
|---|---|---|---|
| 8 | 0 | 0 | 0 |
| 14 | 0 | 0 | 0 |
| 16 | 0 | 1 | 1 |
| 38 | 0 | 0 | 0 |
| 52 | 0 | 0 | 0 |
| 66 | 0 | 0 | 0 |
| 67 | 0 | 0 | 0 |
| 68 | 0 | 0 | 0 |
| 69 | 0 | 0 | 2 |
| 90 | 0 | 1 | 2 |
| 95 | 0 | 0 | 0 |
| 96 | 0 | 0 | 0 |
| 97 | 0 | 1 | 1 |
| 98 | 0 | 1 | 1 |
| 99 | 0 | 0 | 0 |
| 100 | 0 | 0 | 2 |
| 105 | 0 | 0 | 0 |
| 106 | 0 | 0 | 0 |
| 108 | 0 | 0 | 0 |
| 109 | 0 | 0 | 0 |
| 110 | 0 | 0 | 0 |
| Vergleichsmittel A (bekannt) | 2 | 2 | 3 |
| Kontrolle (unbehandelt) | 5 | | |

Beispiel B

Blätter von in Töpfen gewachsenen Weizenkeimlingen der Sorte "Jubilar" werden mit wäßrigen Emulsionen aus 80 % (Gew.%) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltauentwicklung ermittelt.

| Wirkstoff | Befall der Blätter nach Spritzung mit x %iger Wirkstoffbrühe x = 0,025 | 0,012 | 0,006 |
|---|---|---|---|
| 16 | 0 | 0 | 2 |
| 33 | 0 | 0 | 0 |
| 34 | 0 | 2 | 3-4 |
| 36 | 1 | 2 | 2-3 |
| 37 | 0 | 0 | 2-3 |
| 38 | 0 | 0 | 0 |
| 66 | 0 | 0 | 0 |
| 67 | 0 | 2 | 2 |
| 69 | 0 | 1 | 1 |
| 95 | 0 | 0 | 0 |
| 96 | 0 | 0 | 0 |
| 97 | 0 | 0 | 0 |
| 98 | 0 | 0 | 0 |
| 99 | 0 | 0 | 0 |
| 100 | 0 | 0 | 0 |
| 101 | 0 | 0 | 0 |
| 102 | 0 | 0 | 0 |
| 105 | 0 | 0 | 1 |
| 106 | 0 | 0 | 0 |
| 107 | 0 | 0 | 0 |
| 108 | 0 | 0 | 1 |
| 109 | 0 | 0 | 0 |
| 110 | 0 | 0 | 0 |
| 111 | 0 | 0 | 0 |
| 114 | 1 | 2 | 2 |
| 115 | 1 | 2 | 2 |
| Vergleichsmittel A (bekannt | 3 | 4 | 4 |
| Kontrolle (unbehandelt) | 5 | | |

Beispiel C

Blätter von in Töpfen gewachsenen Gurkenkeimlingen werden mit wäßrigen Emulsionen aus 80 % Wirkstoff und 20 % Emulgiermittel besprüht und nach dem Antrocknen des Spritzbelages mit Oidien (Sporen) des Gurkenmehltaus (Erysiphe cicharoiacearum) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

| Wirkstoff | Befall der Blätter nach Spritzung mit 0,025 %iger Wirkstoffbrühe |
|---|---|
| 14 | 0 |
| 16 | 0 |
| 33 | 1 |
| 34 | 1 |
| 36 | 0 |
| 37 | 0 |
| 38 | 0 |
| 66 | 0 |
| 67 | 0 |
| 68 | 0 |
| 69 | 1 |
| 70 | 0 |
| 72 | 0 |
| 73 | 0 |
| 74 | 0 |
| 90 | 0 |
| 91 | 0 |
| 92 | 0 |
| 93 | 0 |
| 94 | 0 |
| 95 | 0 |
| 96 | 0 |
| 97 | 0 |
| 98 | 0 |
| 99 | 0 |
| 100 | 0 |
| 101 | 0 |
| 102 | 0 |
| 105 | 0 |
| 106 | 0 |
| 107 | 0 |
| Kontrolle (unbehandelt) | 5 |

Beispiel D

Blätter von Topfreben der Sorte "Müller-Thurgau" werden mit wäßrigen Emulsionen, die 80 % (Gew.%) des zu prüfenden Wirkstoffs und 20 % Emulgiermittel enthalten, besprüht. Es werden 0,025 %ige Spritzbrühen (bezogen auf die Trockensubstanz) verwendet. Um die Wirkungsdauer der Wirkstoffe beurteilen zu können, werden die Pflanzen nach dem Antrocknen des Spritzbelages zehn Tage im Gewächshaus aufgestellt. Erst dann werden die Blätter mit einer Zoosporenaufschwemmung von Plasmopara viticola (Rebenperonospora) infiziert. Danach kommen die Reben zunächst für 16 Stunden in eine wasserdampfgesättigte Kammer bei 24°C und anschließend 8 Tage in ein Gewächshaus mit Temperaturen zwischen 20 und 30°C. Nach dieser Zeit werden die Pflanzen zur Beschleunigung des Sporangienträgerausbruches abermals während 16 Stunden in der feuchten Kammer aufgestellt. Dann erfolgt die Beurteilung des Ausmaßes des Pilzausbruches auf den Blattunterseiten.

| Wirkstoff | Befall der Blätter nach Spritzung mit 0,025 %iger Wirkstoffbrühe |
|---|---|
| 6 | 0 |
| 34 | 1 |
| 37 | 1 |
| 38 | 1 |
| 52 | 0 |
| 67 | 1 |
| Vergleichsmittel B (bekannt) | 2-3 |
| Kontrolle (unbehandelt) | 5 |

## Beispiel E

Blätter von Topfpflanzen der Sorte "Große Fleischtomate" werden mit wäßrigen Suspensionen, die 0,025 % (Gew.%) Wirkstoff enthalten, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

| Wirkstoff | Befall der Blätter nach Spritzung mit 0,025 %iger Wirkstoffbrühe |
|---|---|
| 6 | 1 |
| 52 | 0 |
| Vergleichsmittel C (bekannt) | 2-3 |
| Kontrolle (unbehandelt) | 5 |

## Patentansprüche

1. Fungizid enthaltend mindestens eine $\gamma$-Azolyl-Verbindung der Formel I

$$R^1-\underset{Az}{CH}-CH_2-\underset{O-R^3}{CH}-R^2 \qquad (I)$$

worin

$R^1$ und $R^2$ gleich oder verschieden sind und Alkyl oder Alkenyl mit bis zu 8 C-Atomen, Cycloalkyl mit bis zu 7 C-Atomen, Furanyl, Thienyl, Naphthyl oder einen gegebenenfalls durch Fluor, Chlor, Brom, Nitro, Trifluormethyl oder Alkyl, Alkoxy oder Alkenyl mit bis zu 8 C-Atomen substituierten Phenylrest bedeuten,

$R^3$ einen gegebenenfalls durch Chlor oder Alkoxy substituierten Alkylrest mit bis zu 10 C-Atomen; einen gegebenenfalls durch Chlor substituierten Alkenylrest mit bis zu 5 C-Atomen; einen Alkinylrest mit bis zu 5 C-Atomen; einen gegebenenfalls durch Fluor, Chlor, Brom, Nitro, Trifluormethyl oder Alkyl, Alkoxy oder Alkenyloxy mit bis zu 4 C-Atomen substituierten Benzylrest; einen $-CO-R^4$-Rest, worin $R^4$ einen gegebenenfalls durch Halogen, Alkoxy, Oxo (= o) oder Carboxyalkyl substituierten Alkylrest mit bis zu 6 C-Atomen oder einen aromatischen Rest bedeutet; oder einen $-CO-NH-R^5$-Rest, worin $R^5$ einen Alkylrest mit bis zu 4 C-Atomen oder einen aromatischen Rest bedeutet, darstellt und

Az einen 1-Imidazolyl-, 1,2,4-Triazolyl- oder 1,2,3- -Triazolylrest bedeutet,

oder deren für Pflanzen verträgliche Salze und Metallkomplexe.

2. Fungizides Mittel gemä Anspruch enthaltend eine γ-Azolyl-Verbindung, ausgewählt aus der Gruppe bestehend aus 1-(4-Chlorphenyl)-1-(1,2,4-triazolyl-(1))-3--acetoxy-4,4-dimethylpentan, 1-(1-Propyloxy)-1-(4--methoxyphenyl)-3-(1,2,4-triazolyl-4)-4,4-dimethylpentan, 1-(1-Butyloxy)-1-(4-methoxyphenyl)-3-(1,2,4--triazolyl-(1))-4,4-dimethylpentan, 1-(1-Pentyloxy)-1--(4-methoxyphenyl)-3-(1,2,4-triazolyl-(1))-4,4-dimethylpentan, 1-(1-Hexyloxy)-1-(4-methoxyphenyl)-3--(1,2,4-triazolyl-(1))-4,4-dimethylpentan, 1-(1-Octyl--oxy)-1-(4-methoxyphenyl)-3-(1,2,4-triazolyl-(1))--4,4-dimethylpentan, 1-Allyloxy-1-(2,4-dichlorphenyl)--3-(1,2,4-triazolyl-(1))-4,4-dimethylpentan, 1-Propargyloxy-1-(2,4-dichlorphenyl)-3-(1,2,4-triazolyl--(1))-4,4-dimethylpentan, 1-Benzyloxy-1-(2,4-dichlorphenyl)-3-(1,2,4-triazolyl-(1))-4,4-dimethylpentan, 1-(2,4-Dichlorphenyl)-1-imidazolyl-3-acetoxy-4,4-dimethylpentan, 1-(2,4-Dichlorphenyl)-1-(1,2,4-triazolyl-(1))-3-allyloxy-4,4-dimethylpentan, 1-(2,4-Dichlorphenyl)-1-(1,2,4-triazolyl-(1))-3-acetoxy-4,4-di methylpentan, 1-(2,4-Dichlorphenyl)-1-(1,2,4-triazoly 1-(1))-3-acetoxy-4-methylhexan, 1-(2-Chlorphenyl)-1--(1,2,4-triazolyl-(1))-3-benzoyl-4-methylhexan, 1-(2,4-Dichlorphenyl)-1-(1,2,4-triazolyl-(1))-3-propionyloxy-4,4-dimethylpentan und 1-(2,4-Dichlorphenyl)-1-(1,2,4-triazolyl-(1))-3-propionyloxy-4--methylhexan.

3. Verfahren zur Bekämpfung von Fungi, dadurch gekennzeichnet, daß man ein Fungizid gemäß Anspruch 1 auf diese einwirken läßt.

4. Verfahren zur vorbeugenden Bekämpfung von Fungi, dadurch gekennzeichnet, daß man mindestens eine Verbindung der Formel I gemäß Anspruch 1 auf durch Fungizid-Befall bedrohte Flächen, Pflanzen oder Saatgüter einwirken läßt.

0031029

Europäisches Patentamt

# EUROPÄISCHER RECHERCHENBERICHT

Nummer der Anmeldung

EP 80 10 7117

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| P/D | EP - A - 0 010 287 (BASF)<br>* Patentansprüche *<br>-- | 1,2 | A 01 N 43/50<br>43/64<br>47/10<br>47/20<br>C 07 D 233/60<br>249/08 |
| A | EP - A - 0 000 940 (BASF) | | |
| A | FR - A - 2 360 583 (BASF)<br>& DE - A - 2 634 511<br>----- | | |
| | | | RECHERCHIERTE SACHGEBIETE (Int. Cl.³)<br>A 01 N 43/50<br>43/64<br>C 07 D 233/60<br>C 07 D 249/08 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Grunden angeführtes Dokument
&: Mitglied der gleichen Patentfamilie, übereinstimmendes Dokument

X Der vorliegende Recherchenbericht wurde für alle Patentanspruche erstellt.

| Recherchenort | Abschlußdatum der Recherche | Prüfer |
|---|---|---|
| Den Haag | 24.03.1981 | DECORTE |